Europäisches Patentamt
European Patent Office
Office européen des brevets

(11) Publication number: **0 181 018**
**A2**

# EUROPEAN PATENT APPLICATION

(21) Application number: 85201632.8

(22) Date of filing: 08.10.85

(51) Int. Cl.[4]: C 07 D 261/14
A 61 K 31/42, A 23 K 1/16

(30) Priority: 01.11.84 GB 8427618

(43) Date of publication of application: 14.05.86 Bulletin 86/20

(84) Designated Contracting States: AT BE CH DE FR GB IT LI LU NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Anderson, Martin
92 Clare Road
Whitstable Kent(GB)

(72) Inventor: Brinnand, Antony Grove
The Meads Perrywood
Selling Faversham Kent(GB)

(72) Inventor: Davis, Royston Henry
76 Bell Road
Sittingbourne Kent(GB)

(74) Representative: Hunter, Keith Roger Ian et al,
4 York Road
London SE1 7NA(GB)

## (54) Anticoccidial isoxazoles and compositions containing them.

(57) Anticoccidial composition comprising an anticoccidially effective amount of a compound of the general formula I

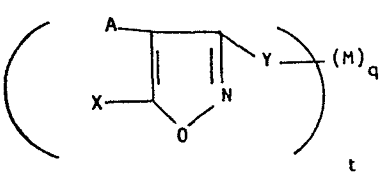

I

in which A represents hydrogen, Cl, Br, I, CN or $NO_2$, X represents a hydrogen atom or an optionally substituted alkyl, aralkyl, aryl, alkaryl, or a heterocyclic group and Y represents a nitrogen-containing heterocyclic group, or a group of general formula II

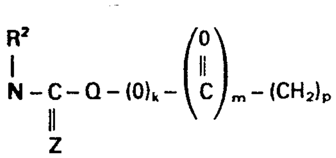

II

in which m is 1 or 0, Z is oxygen or sulphur, Q is hydrogen or

CH in which R is an alkyl group having 1–4 carbon atoms or
|
R

a halogen atom, or Q is $(CH_2)_n$, n being 0–5 or Q is CHHal or $CH_2Hal$ wherein Hal is halogen, $R^2$ is hydrogen or an optionally substituted alkyl or cycloalkyl group, k is 0 or 1, p is 0–5, M is a heterocyclic group containing 1 or more nitrogen atoms, or is halogen, hydrogen, $NHR^1$, $SR^1$, $OR^1$, $NR^1$, $R^1$, N or $N^+$, $R^1$ being an optionally substituted phenyl group, hydrogen or an alkyl group having 1–4 carbon atoms optionally substituted by –OH, –COOH or –COOalkyl, the alkyl group having 1–4 carbon atoms, q being 1 or 0, and t is 1–4 or when t=1 and q=0, Y represents a group of general formula III

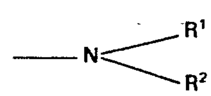

III

./...

EP 0 181 018 A2

in which $R^1$ and $R^2$, which can be the same or different, each represent a hydrogen atom or an optionally-substituted $C_2$–$C_6$ alkyl, cycloalkyl, alkenyl, aryl, alkaryl, aralkyl, acyl, carboxy, alkoxycarbonyl, alkylcarbonyl, dialkoxycarbonyl or sulphonyl group, or in which $R^1$ and $R^2$ together form a ring structure, which including the N atom is prefereably a 5 or 6 membered ring structure or a salt thereof together with a veterinarily acceptable carrier. Poultry feed in which the above anticoccidial composition or anticoccidial compound has been incorporated. Method for preparing such a poultry feed. Method for treating poultry and for rearing birds for human consumption comprising administering thereto the above anticoccidial composition or anticoccidial compound. Process for preparing isoxazoles. Novel isoxazoles.

K 1974 FF

# ANTICOCCIDIAL COMPOSITION, POULTRY FEED, METHOD FOR PREPARING A POULTRY FEED, METHOD FOR TREATING POULTRY, METHOD FOR REARING BIRDS, PROCESS FOR PREPARING ISOXAZOLES AND NOVEL ISOXAZOLES

The present invention relates to anticoccidial compositions comprising certain isoxazoles and their use. It further includes birdfeeds comprising such isoxazoles. Certain of these isoxazoles are novel and are therefore claimed per se..

It is well known that birds, particularly poultry, are susceptible to debilitating infection with coccidia, which can be fatal. A number of avian coccidia are known, and there is a need in the poultry-rearing industry for compounds effective against one or more coccidia. Coccidial infection is a serious problem in poultry husbandry, particularly when the birds are kept under "broiler house" conditions. It has now been found that certain isoxazoles are capable of exhibiting good anti-coccidial properties against Eimeria tenella, Eimeria acervulina, Eimeria maxima, Eimeria brunetti and Eimeria necatrix , when administered orally to poultry like chickens and turkeys at appropriate dose levels.

Accordingly the present invention provides an anticoccidial composition which comprises an anticoccidially effective amount of a compound of the general formula (I):

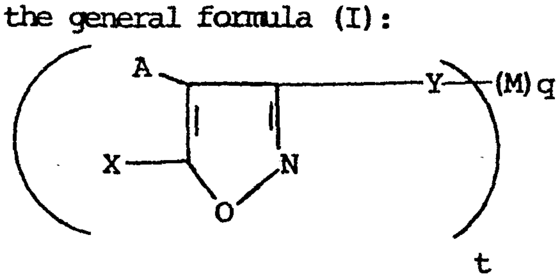

(I)

,in which A represents hydrogen, Cl, Br, I, CN or $NO_2$, X represents a hydrogen atom or an optionally substituted alkyl, aralkyl, aryl, alkaryl, or a heterocyclic group and Y represents an amino or substituted amino group, or a nitrogen-containing heterocyclic group, or a group of general formula II

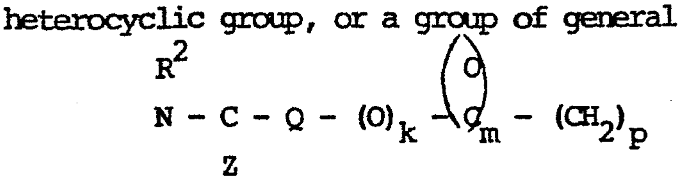

(II)

in which m is 1 or 0, Z is oxygen or sulphur, Q is hydrogen or CH
|
R, in which R is an alkyl group having 1-4 carbon atoms or a halogen atom, or Q is $(CH_2)_n$, n being 0-5 or Q is CH Hal or $CH_2Hal$ wherein Hal is halogen, $R^2$ is hydrogen or an optionally substituted alkyl or cycloalkyl group, k is 0 or 1, p is 0-5, M is a heterocyclic group containing 1 or more nitrogen atoms, or is halogen, hydrogen, $NHR^1$, $SR^1$, $OR^1$, $NR^1$, $R^1$, N or $N^+$, $R^1$ being an optionally substituted phenyl group, hydrogen or an alkyl group having 1-4 carbon atoms optionally substituted by -OH, -COOH or -COOalkyl, the alkyl group having 1-4 carbon atoms, q being 1 or 0, and t is 1-4 or when t=1 and q=0 Y represents a group of general formula III and M and q having the meaning as defined before

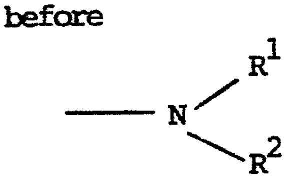

(III)

, in which $R^1$ and $R^2$, which can be the same or different, each represent a hydrogen atom or an optionally-substituted $C_2$-$C_6$ alkyl, cycloalkyl, alkenyl, aryl, alkaryl, aralkyl, acyl, carboxy, alkoxycarbonyl, alkylcarbonyl, dialkoxycarbonyl or sulphonyl group, or in which $R^1$ and $R^2$ together form a ring structure, which including the N atom is preferably a 5 or 6 membered ring structure or a salt thereof together with a veterinarily acceptable carrier.

Preferably the anticoccidial composition comprises a compound of formula I in which Y is a group of general formula

II in which $R^2$ is an alkyl group having 1-4 carbon atoms, Z is oxygen, Q is $(CH_2)_n$ wherein n is 0-3, k=0, m=1, p=o, q=1, t=1 and M is OH.

Another preferred anticoccidial composition comprises a compound of formula I in which X represents an aralkyl, aryl, alkaryl, or a substituted heterocyclic group, of which at least one of the substituents is an electron withdrawing group.

More preferred the anticoccidial composition comprises a compound of formula I in which X represents a phenyl group substituted by at least chlorine or bromine. Still more preferred is an anticoccidial composition which comprises a compound of formula I in which X is p-chlorophenyl and Y is

$N-COCH_2COOH$ or $N-COCOOH$
$\quad |\qquad\qquad\qquad\quad |$
$C_2H_5\qquad\qquad\quad C_2H_5$

Said composition is preferably of the nature of the conventional kind of poultry premix save that the active anticoccidial component is a compound of formula I or a salt form thereof; and such a premix may contain other additives, for example, vitamins, minerals, antibiotics and anti-oxidants as is normal practice; the concentration of anticoccidial (and other additives, if present) being such that an effective dose is provided by mixing of the order of 0.5% wt of the premix with the base feedstuff. The feedstuff for sale to the poultry farmer can be made in accordance with conventional practice by mixing the premix thoroughly with the base feedstuff, using an indicator to monitor samples taken during the mixing/grinding operation to ensure the required quality of mixing is achieved, followed by coagulation with a starch additive at about 70°C to produce a granular feed product.

Therefore the present invention also includes a feed suitable for a bird, particularly a bird, for example a chicken or a turkey, intended for human consumption, which contains a compound of formula I, and a method of making such a feed by mixing with, or otherwise incorporating in, bird-edible material a compound of formula I preferably by adding to that material a

premix as referred to above. The present invention also includes a method for treating poultry infected by coccidia in order to prevent the premature mortality or physical deterioration of the poultry. The method is particularly useful to treat chickens and turkeys, intended for human consumption. The method comprises administering thereto, preferably in a mixture with its feed, a compound of formula I or a composition as defined above. The present invention also includes a method of rearing birds, for example chickens or turkeys, for ultimate human consumption, which comprises orally administering thereto a compound of formula I, or a composition as defined above or a feed as defined above.

The present invention also includes processes for preparing the at present preferred isoxazoles of formula I in which X is a halo-substituted phenyl radical and Y is a mono-or di-alkylamino radical such as a group of general formula II.

The isoxazoles in which Y is a mono-alkylamino radical can be prepared by reacting a halo-substituted acetophenone with an alkyl isothiocyanate and methyl iodide in the presence of sodium hydride to form an intermediate of general formula:

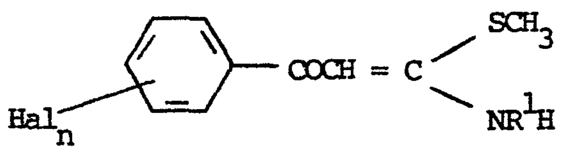

, which is thereafter converted to the isoxazole by reaction with hydroxylamine.

The isoxazoles in which Y is a di-alkylamino radical can be prepared from a halo-substituted acetophenone by forming a similar intermediate of general formula:

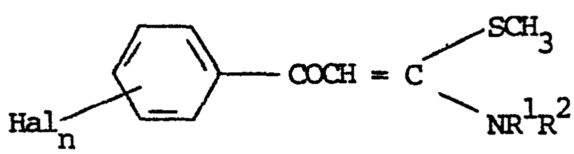

by a two-stage reaction comprising first reacting the halo-substituted acetophenone with carbon disulphide and methyl iodide in the presence of sodium hydride and then reacting the product with a di-alkylamine. The resulting intermediate is

thereafter converted to the isoxazole by reaction with hydroxylamine.

The following examples further illustrate the present invention.

Example I

3-ethylamino - 5- (4'-chlorophenyl) isoxazole (No. 3 in Table I)

1-(4'-chlorophenyl)-3-ethylamino-3-methylthioprop-2-en-1-one (26g), and hydroxylamine hydrochloride (10g) in pyridine (15ml) were heated together under reflux for 3-4 hours. The solvent was then removed under reduced pressure and the residue dissolved in chloroform. The chloroform after washing with water was removed and the residue crystallised from methanol to give the required 3-ethylamino-5-(4'-chlorophenyl) isoxazole (mpt. 134-135°C).

Analysis

| | | | |
|---|---|---|---|
| Calculated for $C_{11}H_{11}ON_2cl$ | C 59.3 | H 5.0 | N 12.6 |
| found | C 59.2 | H 5.0 | N 12.5 |

Example II

3-diethylamino-5-(4'-bromophenyl) isoxazole (No. 15 in Table I)

1-(4'-bromophenyl)-3,3-diethylamino-3-methylthioprop-2-en-1-one bromophenyl (10g) and hydroxylamine hydrochloride (0.5g) in pyridine (15ml) were heated under reflux for 3-4 hours. The solvent was then removed under reduced pressure and the residue dissolved in diethylether. The diethylether after washing with water was removed and the residue was purified by chromatography on silica gel using chloroform as eluent to give the required 3-diethylamino-5-(4'-bromophenyl) isoxazole (m.pt 75-76°C).

Analysis

| | | | |
|---|---|---|---|
| Calculated for $C_{13}H_{15}ON_2Br$ | C 52.9 | H 5.1 | N 9.5 |
| found | C 52.8 | H 5.8 | N 9.4 |

Example III

3-[ethylamino(N-carboxyethanoyl)]-5-(4'chlorophenyl)isoxazole (No. 22 in Table I)

3-ethylamino-5-(4'-chlorophenyl)isoxazole (25g) (as

prepared according to Example I) and ethylmalonylchloride technical (20g) and triethylamine (20g) in chloroform were treated under reflux for 1½ hours.

The 3-[ethylamino-(N-carboxyethanoyl)]-5-(4'chlorophenyl) isoxazole was hydrolysed to the required acid by dissolving the ester in acetone (150mls) adding $K_2CO_3$ (15g) and stirring at 55°C for 14 hours. The solvent was removed and residue dissolved in water and acidified with 2N HCl. The acid obtained was then filtered and dried (12g).

Example IV

3-ethylamino-4-chloro-5-(4'chlorophenyl)isoxazole

(No. 29 in Table I)

Dichloromethane solution (100 ml) of chlorine (1.5g) added to 3-ethylamino-5-(4'chlorophenyl)isoxazole (4.4g) in dichloromethane (100 ml) at 10°C.

The dichloromethane solution after washing with 5% potassium carbonate solution was removed under reduced pressure and the residue crystallised from methanol to give the required 3-ethylamino-4-chloro-5-(4'chlorophenyl)isoxazole (4.6g) mpt. 127°C.

Analysis

| | | | |
|---|---|---|---|
| Calculated for $C_{11}H_{10}ON_2Cl_2$ | C 51.4 | H 3.9 | N 10.9 |
| Found | C 51.2 | H 3.8 | N 10.8 |

Example V

3-ethylamino-4-cyano-5(4'chlorophenyl)isoxazole

(No. 30 in Table I)

1-(4'chlorophenyl)-2-cyano-3-ethylamino-3-methylthio prop-2-en-1-one (6.5g) and hydroxylamine (1.4g) in pyridine (50ml) water (4ml) was kept at 0°C for 24 hours. The solvent was removed under reduced pressure and the residue dissolved in chloroform. The chloroform after washing with water was removed and the residue was purified by chromatography on silica gel using hexane-ether 1:2 as eluent to give 3-ethylamino-4-cyano-5(4'chlorophenyl)isoxazole mpt. 183°C.

Analysis

| | | | |
|---|---|---|---|
| Calculated for $C_{12}H_{10}ON_3Br$ | C 49.3 | H 3.45 | N 14.4 |
| Found | C 49.4 | H 3.3 | N 14.2 |

Example VI

3[N-2' (bromoethanoyl)-N-ethylamino]-5(4"chlorophenyl) isoxazole

(No. 31 in Table I)

3-ethylamino-5-(4'chlorophenyl)isoxazole (22.2g) and bromoacetylbromide (10.1g) refluxed in chloroform for 2 hours. The solution was cooled and triethylamino (50.0g) added. The chloroform solution after washing with water was removed under reduced pressure and the residue crystallised from hexane to give required 3[N-2'bromoethanoyl)-N-ethylamino]-5(4"chlorophenylisoxazole (34g) mpt 74°C.

Analysis

| | | | |
|---|---|---|---|
| Calculated for $C_{13}H_{12}N_2O_2ClBr$ | C 45.4 | H 3.5 | N 8.2 |
| Found | C 45.8 | H 3.5 | N 8.1 |

Example VII

3[N-2'ethylamino ethanoyl)-N-ethylamino]-5(4"chloro phenyl) isoxazole

(No. 32 in Table I)

A ether solution of 3[N-2'bromoethanoyl)N-ethylamino]-5-(4"chlorophenyl)isoxazole (5.1g) as prepared according to Example VI was added to ethylamino (10ml) in ether (100ml). After 1 hour the ether mixture after having been washed with 5% potassium carbonate solution was removed under reduced pressure and the residue recrystallised from hexane to give the required 3-[N-2'ethylaminoethanoyl)-N-ethylamino]-5(4"chlorophenyl) isoxazole (4.1g) mpt 84°C.

Analysis

| | | | |
|---|---|---|---|
| Calculated for $C_{15}H_{18}O_2N_3Cl$ | C 58.5 | H 5.9 | N 13.7 |
| Found | C 58.7 | H 5.4 | N 13.6 |

Example VIII

3-[N-(2'-chloroethanoyl)-N-ethylamino]-5-(4"-chlorophenyl)-isoxazole

(No. 33 in Table I)

Potassium carbonate (1.0g; 7.25mmol) in water (10cm$^3$) was

added dropwise, with stirring, to a solution of (±) 3-[N-(2'-chloro-3'-ethoxy-propan-1',3'-dioyl)-N-ethylamino[-5-(4"-chlorophenyl)isoxazole (2.5g; 6.74mmol) in ethanol ($20cm^3$) at room temperature. The mixture was heated to 45°C for 3 hours, allowed to cool, and stood at room temperature for 15 hours. The solvent was then removed as far as practicable, on a rotary evaporator, the residues then washed with water, extracted into $CHCl_3$, and chromatographed (silica/$CH_2Cl_2$). The product yield was 1.2g (59.5%).

Analysis

| | | | |
|---|---|---|---|
| Calculated $C_{13}H_{12}N_2O_2Cl_2$; | C 52.2 | H 4.0 | N 9.4 |
| Found | C 52.2 | H 3.9 | N 9.2 |

Example IX

3-[N-(2',2'-dichloro-3'-ethoxy-propan-1',3'-dioyl)-N-ethylamino]-5-(4"-chlorophenyl)isoxazole

(No. 34 in Table I)

Chlorine gas (0.93g; 13.1mmol) in carbon tetrachloride ($21.5m^3$) was added dropwise, with stirring, to a solution of 5-(4'-chlorophenyl)-3-[N-(3"-ethoxypropan-1",3"-dioyl)-N-ethylamino]isoxazole (4.4g; 13.1mmol) in carbon tetrachloride ($40cm^3$), and stirred at room temperature for 1 hour. The solvent was then removed and the residual oil eluted down a silica/$CH_2Cl_2$ column. This yielded 0.9g (17.0%) of the product.

Analysis

| | | | |
|---|---|---|---|
| Calculated $C_{16}H_{15}N_2O_4Cl_3$; | C 47.3 | H 3.7 | N 6.9 |
| Found | C 47.1 | H 3.7 | N 6.8 |

Example X

(±)3-[N-(2'-chloro-3'-ethoxy-propan-1',3'-dioyl)-N-ethylamino]-5-(4"-chlorophenyl)isoxazole

(No. 35 in Table I)

Chlorine gas (0.93g; 13.1mmol) in carbon tetrachloride ($21.5cm^3$) was added dropwise, with stirring, to a solution of 5-(4'-chlorophenyl)-3-[N-(3"-ethoxypropan-1",3"-dioyl)-N-ethylamino]isoxazole (4.4g; 13.1mmol) in carbon tetrachloride ($40cm^3$), and stirred at room temperature for 1 hour. The solvent was then removed and the residual oil eluted down a silica/$CH_2Cl_2$ column. This afforded 3.7g (76.3%) of the above product.

Analysis

| | | | | |
|---|---|---|---|---|
| Calculated | $C_{16}H_{16}N_2O_4Cl_2$; | C 51.8 | H 4.3 | N 7.5 |
| Found | | C 51.6 | H 4.3 | N 7.4 |

Example XI

3-[N-(2'-acetoxyethanoyl)-N-ethylamino]-4-chloro-5-(4"-chlorophenyl)isoxazole

Chlorine gas (0.5g; 7.04mmol) in carbon tetrachloride ($5.6cm^3$) was added dropwise, with vigorous stirring, to a solution of 3-[N-(2'-acetoxyethanoyl)-N-ethylamino]-5-(4"-chlorophenyl)isoxazole (1.1g; 3.41mmol) in carbon tetrachloride ($25cm^3$) and the mixture stirred at room temperature for 30hours. The solvent was removed and the residual oil chromatographed [silica/diethylether: petroleum ether (1:1)] yielding 1.0g (82.1%) of product as an oil.

Analysis

| | | | | |
|---|---|---|---|---|
| Calculated | $C_{15}H_{14}N_2O_4Cl_2$; | C 50.4 | H 3.9 | N 7.8 |
| Found | | C 50.8 | H 4.1 | N 7.5 |

Example XII

3-(N-ethylamino-5-(4'-trifluoromethylphenyl)isoxazole (No. 37 in Table I)

3-(N-ethylamino)-1-(4'-trifluoromethylphenyl)-3-methylthio prop-2-en-1-one (2.8g; 9.7mmol) in dry pyridine ($30cm^3$) was treated with hydroxylamine hydrochloride (1.0g; 14mmol) at 60°C for 2½ hours. The product was chromatographed (silica/$CH_2CL_2$) yielding 2.2g (88.7%).

Analysis

| | | | | |
|---|---|---|---|---|
| Calculated | $C_{12}H_{11}N_2OF_3$ | C 56.3 | H 4.3 | N 10.9 |
| Found | | C 56.5 | H 4.2 | N 10.9 |

Example XIII

3-[N-2'-acetoxyethanoyl)-N-ethylamino]-5(4"chlorophenyl)-isoxazole

(No. 38 in Table I)

3-[N-2'bromoethanoyl)N-ethylamino]-5(4"chlorophenyl) isoxazole (1g) refluxed in acetic acid (30ml) with sodium acetate (1g) for 4 hours. The mixture was poured onto ice-water and extracted with ether. The ether was removed under reduced pressure and residue was purified by chromatography on silica gel using chloroform as eluent to give

3-[N-2'acetoxyethanoyl)N-ethylamino]-5

(4"chlorophenyl)isoxazole (0.6g) mpt 82°C

Analysis

| | | | | |
|---|---|---|---|---|
| Calculated | $C_{15}H_{15}N_2O_4Cl$ | C 55.8 | H 4.7 | N 8.7 |
| Found | | C 55.7 | H 4.5 | N 8.5 |

In Table I examples of compounds are shown which have been prepared according to the process as described before.

Table II gives details of the test results obtained in the in vivo testing of three typical isoxazoles which can be used in carrying out the present invention. The tests have been indicated as Exp. 1, Exp. 2, Exp. 3 and Exp. 4 Chickens were infected orally with a quantity of sporulated Eimeria tenella (Weybridge strain) Oöcycsts the sizes of inoculum used being 50,000, 75,000 and 100,000. The isoxazole content of the broiler feed being either 125 ppm or 250 ppm. The results given in the table are those obtained with uninfected, untreated control birds and those obtained with infected birds treated either with the solvent ("placebo") or with the specified isoxazole or with one or other of the two commercially available anticoccidials, "Nicarbazin" and "Monensin"

Table III gives details of test results obtained in the in vivo testing of 3-[ethylamino(N-carboxyethanoyl)]-5-(4'-chlorophenyl)isoxazole (compound 22) against Eimeria tenella and Eimeria necatrix.

Table IV gives results of an in vitro evaluation of certain compounds against E. tenella.

Table I

| No | Compound | Melting Point °C | Analysis | | C | H | N |
|---|---|---|---|---|---|---|---|
| 1 | 3-methylamino-5-(4'-chlorophenyl)isoxazole | 172-4 | Calculated | $C_{10}H_9ON_2Cl$ | 57.6 | 4.3 | 13.4 |
| | | | Found | | 57.4 | 4.4 | 13.4 |
| 2 | 3-methylamino-5-(2',5'-dichlorophenyl)-isoxazole | 160 | | $C_{10}H_8ON_2Cl_2$ | 49.4 | 3.3 | 11.5 |
| | | | | | 49.2 | 3.2 | 11.6 |
| 3 | 3-ethylamino-5-(4'-chlorophenyl)isoxazole | 134-135 | | $C_{11}H_{11}ON_2Cl$ | 59.3 | 5.0 | 12.6 |
| | | | | | 59.2 | 5.0 | 12.5 |
| 4 | 3-[methylamino(N-acetyl)]-5-(4'-chlorophenyl)isoxazole | 118-119 | | $C_{12}H_{11}O_2N_2Cl$ | 57.5 | 4.4 | 11.2 |
| | | | | | 57.6 | 4.2 | 11.3 |
| 5 | 3-cyclopropylamino-5-(4'-chlorophenyl)-isoxazole | 124-125 | | $C_{12}H_{11}ON_2Cl$ | 61.4 | 4.7 | 11.9 |
| | | | | | 61.0 | 4.7 | 11.9 |
| 6 | 3-methylamino-5-(4'-bromophenyl)isoxazole | 167 | | $C_{10}H_9ON_2Br$ | 47.4 | 3.6 | 11.1 |
| | | | | | 47.2 | 3.4 | 11.1 |
| 7 | 3-piperidyl -5-(4'-chlorophenyl)isoxazole | 137-138 | | $C_{14}H_{15}ON_2Cl$ | 64.0 | 5.7 | 10.7 |
| | | | | | 63.7 | 5.5 | 10.6 |
| 8 | 3-methylamino-5-phenyl isoxazole | 84-85 | | $C_{10}H_{10}ON_2$ | 68.9 | 5.8 | 16.1 |
| | | | | | 69.1 | 5.8 | 16.2 |
| 9 | 3-methylamino-5-(4'-methylphenyl)isoxazole | 115-116 | | $C_{11}H_{12}ON_2$ | 70.2 | 6.4 | 14.9 |
| | | | | | 70.0 | 6.3 | 14.7 |

TABLE I (continued)

| No | Compound | Melting Point °C | Analysis | C | H | N |
|---|---|---|---|---|---|---|
| 10 | 3-dimethylamino-5-(4'-chlorophenyl)isoxazole | 120–122 | $C_{11}H_{11}ON_2Cl$ | 57.3 | 3.0 | 12.0 |
| | | | | 58.8 | 4.9 | 12.5 |
| 11 | 3-N-morpholinyl-5-(4'-bromphenyl)isoxazole | 75 | $C_{13}H_{13}N_2O_2Br$ | 50.5 | 4.2 | 9.1 |
| | | | | 50.8 | 4.0 | 9.1 |
| 12 | 3-[methylamino(N-methylsulphonyl)]-5-(4'-chlorophenyl)isoxazole | 182–3 | $C_{11}H_{11}N_2O_3SCl$ | 46.1 | 3.9 | 9.8 |
| | | | | 46.2 | 4.0 | 9.8 |
| 13 | 3-methylamino-5-(4'-flurophenyl)isoxazole | 143–144 | $C_{10}H_9N_2OF$ | 62.5 | 4.7 | 14.6 |
| | | | | 62.0 | 4.8 | 14.5 |
| 14 | 3-Ethylamino-5-(4'-bromophenyl)isoxazole | 135–136 | $C_{11}H_{11}ON_2Br$ | 49.5 | 4.1 | 10.5 |
| | | | | 49.6 | 4.2 | 10.5 |
| 15 | 3-diethylamino-5-(4'-bromophenyl)isoxazole | 75–76 | $C_{13}H_{15}ON_2Br$ | 52.9 | 5.1 | 9.5 |
| | | | | 52.8 | 5.3 | 9.4 |
| 16 | 3-[methylamino(N-acetyl)]-5-(4'-bromophenyl)isoxazole | 140–141 | $C_{12}H_{11}O_2N_2Br$ | 48.8 | 3.8 | 9.5 |
| | | | | 48.5 | 3.7 | 9.4 |
| 17 | 3-methylamino-5-(2'-chlorophenyl)isoxazole | 75–76 | $C_{10}H_9ON_2Cl$ | 57.6 | 4.3 | 13.4 |
| | | | | 57.3 | 4.5 | 13.3 |

TABLE I (continued)

| No | Compound | Melting Point °C | Analysis | C | H | N |
|---|---|---|---|---|---|---|
| 18 | 3-methylamino-5-(3'-bromphenyl)isoxazole | 91-92 | $C_{10}H_9ON_2Br$ | 47.4 | 3.6 | 11.1 |
| | | | | 47.1 | 3.5 | 10.6 |
| 19 | 3-methylamino-5-(3,4-dichlorophenyl)isoxazole | 95 | $C_{10}H_8ON_2Cl_2$ | 49.4 | 3.3 | 11.5 |
| | | | | 49.9 | 3.2 | 10.9 |
| 20 | 3-allylamino-5-(4'-bromophenyl)isoxazole | 126 | $C_{12}H_{11}ON_2Br$ | 51.6 | 4.0 | 10.0 |
| | | | | 50.4 | 3.9 | 10.0 |
| 21 | 3-[ethylamino(N-carboxy carbonyl)]-5-(4'-chlorophenyl)isoxazole | 129 | $C_{13}H_{11}O_4N_2Cl$ | 53.0 | 3.7 | 9.5 |
| | | | | 52.6 | 3.6 | 9.3 |
| 22 | 3-[ethylamino(N-carboxyethanoyl)]-5-(4'-chlorophenyl)isoxazole | 142 | $C_{14}H_{13}O_4N_2Cl$ | 54.5 | 4.2 | 9.1 |
| | | | | 54.4 | 4.0 | 9.0 |
| 23 | 5-(4'chlorophenyl-3-[N-ethyl, N-2-methoxy ethanoamide]isoxazole | 107 | $C_{14}H_{15}N_2O_3Cl$ | 57.05 | 5.1 | 9.5 |
| | | | | 56.9 | 5.0 | 9.3 |
| 24 | 5-(4'chlorophenyl)-3-[N-ethyl,N-2-methyl amino ethanoamide]isoxazole | 113 | $C_{14}H_{16}N_3O_2Cl$ | 57.2 | 5.5 | 14.3 |
| | | | | 57.3 | 5.5 | 14.2 |
| 25 | 5-(4'chlorophenyl)-3-[N-ethyl-2-imidazol-yl ethanoamide]isoxazole | 166 | $C_{16}H_{15}O_2N_4Cl$ | 58.3 | 4.6 | 17.0 |
| | | | | 58.4 | 4.5 | 16.7 |

TABLE I (continued)

| No | Compound | Melting Point °C | Analysis | C | H | N |
|---|---|---|---|---|---|---|
| 26 | 5-(4'chlorophenyl)-3-(N-ethyl,N-formyl)isoxazole | 94 | $C_{12}H_{11}O_2N_2Cl$ | 57.5 | 4.4 | 11.2 |
| | | | | 57.8 | 4.3 | 11.05 |
| 27 | 5-(4'chlorophenyl)-3-[N-ethyl-2-(4'nitrophenoxy) ethanoamide]isoxazole | 148 | $C_{19}H_{16}O_5N_3Cl$ | 56.8 | 4.0 | 10.5 |
| | | | | 56.7 | 3.6 | 10.2 |
| 28 | 5-(4'chlorophenyl)-3-[N-ethyl-N-(N'-2-ethano-amido-N-ethanoic acid]isoxazole hydrochloride | 245 | $C_{15}H_{17}O_4N_3Cl_2$ | 48.1 | 4.6 | 11.2 |
| | | | | 48.4 | 4.5 | 11.1 |
| 29 | 3-ethylamino-4-chloro-5-(4'chlorophenyl) isoxazole | 127 | $C_{11}H_{10}ON_2Cl_2$ | 51.4 | 3.9 | 10.9 |
| | | | | 51.2 | 3.8 | 10.8 |
| 30 | 3-ethylamino-4-cyano-5-(4'chlorophenyl) isoxazole | 183 | $C_{12}H_{10}ON_3Br$ | 49.3 | 3.5 | 14.4 |
| | | | | 49.4 | 3.3 | 14.2 |
| 31 | 3-[N-2'bromoethanoyl)-N-ethylamino]-5(4" chlorophenyl isoxazole | 74 | $C_{13}H_{12}O_2N_2ClBr$ | 45.4 | 3.5 | 8.2 |
| | | | | 45.8 | 3.5 | 8.1 |
| 32 | 3-[N-2'ethylaminoethanoyl)N-ethylamino]-5-(4"chlorophenyl)isoxazole | 84 | $C_{15}H_{18}O_2N_3Cl$ | 58.5 | 5.9 | 13.7 |
| | | | | 58.7 | 5.4 | 13.6 |

TABLE I (continued)

| No | Compound | Melting Point °C | Analysis | C | H | N |
|---|---|---|---|---|---|---|
| 33 | 3-[N-(2'chloroethanoyl)-N-ethylamino]5-(4"chloro phenyl)isoxazole | oil | $C_{13}H_{12}N_2O_2Cl_2$ | 52.2<br>52.2 | 4.0<br>3.9 | 9.4<br>9.2 |
| 34 | 3-[N-(2',2'dichloro-3'ethoxypropan-1',3'dioyl)N-ethylamino]-5-(4"chlorophenyl)isoxazole | oil | $C_{16}H_{15}N_2O_4Cl_3$ | 47.3<br>47.1 | 3.7<br>3.7 | 6.9<br>6.8 |
| 35 | 3-[N-2'chloro-3'ethoxy-propan 1',3dioyl)N-ethyl-amino]5-(4"chlorophenyl)isoxazole | oil | $C_{16}H_{16}N_2O_4Cl_2$ | 51.8<br>51.6 | 4.3<br>4.3 | 7.5<br>7.4 |
| 36 | 3-[N-(2'acetoxyethanoyl)-N-ethylamino]-4-chloro-5-(4"chlorophenyl)isoxazole | oil | $C_{15}H_{14}N_2O_4Cl_2$ | 50.4<br>50.8 | 3.9<br>4.1 | 7.8<br>7.5 |
| 37 | 3-(N-ethylamino)-5-(4'trifluoromethyl)isoxazole | | $C_{12}H_{11}N_2OF_3$ | 56.3<br>56.5 | 4.3<br>4.2 | 10.9<br>10.9 |
| 38 | 3-[N-2'acetoxyethanoyl)-N-ethylamino]-5-(4"chloro phenyl)isoxazole | 82 | $C_{15}H_{15}N_2O_4Cl$ | 55.8<br>55.7 | 4.7<br>4.5 | 8.7<br>8.5 |

TABLE I (continued)

| No | Compound | Melting Point °C | Analysis | C | H | N |
|---|---|---|---|---|---|---|
| 39 | 3-[N-ethylamino N-formyl] 4-chloro-5(4'chloro phenyl)isoxazole | 53 | $C_{12}H_{10}O_2N_2Cl_2$ | 50.6<br>50.4 | 3.5<br>3.5 | 9.8<br>9.8 |
| 40 | 3-[N-ethylamino N acetyl] 4-chloro-5(4'chloro phenyl)isoxazole | 81 | $C_{13}H_{12}O_2N_2Cl_2$ | 52.2<br>52.4 | 4.0<br>4.1 | 9.4<br>9.3 |
| 41 | 3-[N-(2'bromoethanoyl) N-ethylamino]4-chloro-5 (4"chlorophenyl)isoxazole | 125 | $C_{13}H_{11}O_2N_2Cl_2Br$ | 41.3<br>41.4 | 2.9<br>3.0 | 7.4<br>7.7 |
| 42 | 3][N-2'methylaminoethanoyl)N-ethylamino]4-chloro-5(4"chlorophenyl)isoxazole | 69 | $C_{14}H_{15}O_2N_3Cl_2$ | 51.2<br>51.6 | 4.6<br>4.6 | 12.8<br>12.8 |
| 43 | 3-ethylamino 4-bromo-5(4'chlorophenyl)isoxazole | 109 | $C_{11}H_{10}ON_2ClBr$ | 43.8<br>44.3 | 3.3<br>3.4 | 9.3<br>9.5 |
| 44 | 3-ethylamino-4-bromo-5(4'methoxyphenyl)isoxazole | 102 | $C_{12}H_{13}O_2N_2Br$ | 48.5<br>48.6 | 4.4<br>4.4 | 9.4<br>9.5 |

TABLE I (continued)

| No | Compound | Melting Point °C | Analysis | C | H | N |
|---|---|---|---|---|---|---|
| 45 | 3-[N-(2'bromoethanoyl)-N-ethylamino]-5-(4"bromo phenyl)isoxazole | 94 | $C_{13}H_{12}O_2N_2Br_2$ | 40.2<br>40.4 | 3.1<br>3.1 | 7.2<br>7.3 |
| 46 | 3-ethylamino-4-bromo-5(4bromophenyl)isoxazole | 120 | $C_{11}H_{10}ON_2Br_2$ | 38.2<br>38.3 | 2.9<br>2.9 | 8.1<br>8.0 |
| 47 | 3-N-(2'acetoxyethanoyl)N-ethylamino]5-(4"bromo phenyl)isoxazole | 114 | $C_{15}H_{15}O_4N_2Br$ | 49.1<br>49.0 | 4.1<br>4.1 | 7.6<br>7.7 |
| 48 | 3-[N-(2'bromoethanoyl)N-ethylamino]4-bromo-5(4"chlorophenyl)isoxazole | 143 | $C_{13}H_{11}O_2N_2Br_2Cl$ | 37.0<br>37.4 | 2.6<br>2.6 | 6.6<br>6.7 |
| 49 | 3-[N-2'fluoroacetoxyethanoyl)N-ethylamino]5(4" chlorophenyl)isoxazole | 110 | $C_{15}H_{14}N_2O_4ClF$ | 52.9<br>52.9 | 4.1<br>4.2 | 8.2<br>8.2 |
| 50 | 3-[N-acetyl N-ethylamino]4-bromo, 6(4"chloro phenyl)isoxazole | 106 | $C_{13}H_{12}N_2O_2ClBr$ | 45.3<br>45.4 | 3.5<br>3.4 | 8.1<br>8.2 |

TABLE I (continued)

| No | Compound | Melting Point °C | Analysis | C | H | N |
|---|---|---|---|---|---|---|
| 51 | 3-[N-(2'methylaminoethanoyl)N-ethylamino]4-bromo 5(4"chlorophenyl)isoxazole | 84 | $C_{14}H_{15}N_3O_2ClBr$ | 45.1<br>45.0 | 4.1<br>4.1 | 11.3<br>11.2 |
| 52 | 3-[N-ethylamino, N-formyl]4-bromo 5(4"chloro phenyl)isoxazole | 67 | $C_{12}H_{10}O_2N_2ClBr$ | 43.7<br>43.6 | 3.1<br>3.0 | 8.5<br>8.5 |
| 53 | 3-[N-2'bromoethanoyl)N-ethylamino]4-bromo 5(4"bromophenyl)isoxazole | 150 | $C_{13}H_{11}N_2O_2Br_3$ | 33.4<br>33.5 | 2.4<br>2.5 | 6.0<br>6.2 |
| 54 | 3-[N-(2'acetoxyethanoyl)N-ethylamino]4-bromo 5(4"chlorophenyl)isoxazole | 60 | $C_{15}H_{14}N_2O_4BrCl$ | 44.9<br>45.0 | 3.5<br>3.6 | 4.0<br>7.0 |
| 55 | 3-[N(2'methylaminoethanoyl)N-ethylamino] 5(4"bromophenyl)isoxazole | 116 | $C_{14}H_{16}N_3O_2Br$ | 49.7<br>49.8 | 4.8<br>4.8 | 12.4<br>12.5 |
| 56 | 3-[N-trichloroacetyl N-ethylamino]5-(4'chloro phenyl)isoxazole | 99 | $C_{13}H_{10}N_2O_2Cl_4$ | 42.4<br>42.5 | 2.7<br>2.8 | 7.6<br>7.6 |

TABLE I (continued)

| No | Compound | Melting Point °C | Analysis | C | H | N |
|---|---|---|---|---|---|---|
| 57 | 3-[N-(2'acetoxyethanoyl)N-ethylamino]-4chloro 5-(4"bromophenyl) isoxazole | 47 | $C_{15}H_{14}N_2O_4ClBr$ | 44.8<br>44.9 | 3.5<br>3.8 | 7.0<br>7.2 |
| 58 | 3-[N-(2'acetoxyethanoyl)N-ethylamino]-4bromo 5-(4"bromophenyl) isoxazole | 65 | $C_{15}H_{14}N_2O_4Br_2$ | 40.4<br>40.5 | 3.2<br>3.1 | 6.3<br>6.3 |
| 59 | 3-[N-(2'ethylaminoethanoyl)N-ethylamino]-4-bromo 5-(4"chlorophenyl) isoxazole | 62 | $C_{15}H_{17}N_3O_2ClBr$ | 46.6<br>46.6 | 4.4<br>4.5 | 10.9<br>11.1 |
| 60 | 3-[N-ethylamino] 4-chloro-5(4'bromophenyl) isoxazole | 144 | $C_{11}H_{10}ON_2ClBr$ | 43.8<br>43.7 | 3.3<br>3.3 | 9.3<br>9.3 |
| 61 | 3-[N(2'acetoxy thioethanoyl)N-ethylamino]5-(4'chlorophenyl) isoxazole | 106 | $C_{15}H_{15}N_2O_3SCl$ | 53.2<br>53.2 | 4.4<br>4.4 | 8.3<br>8.4 |
| 62 | 3-[N(2'acetoxy propanoyl)N-ethylamino]5-(4' chlorophenyl) isoxazole | 70 | $C_{16}H_{17}N_2O_4Cl$ | 57.0<br>57.2 | 5.1<br>4.7 | 8.3<br>8.5 |

TABLE I (continued)

| No | Compound | Melting Point °C | Analysis | C | H | N |
|---|---|---|---|---|---|---|
| 63 | 3-[N(2'acetoxy chloroethanoyl)N-ethylamino] 5-(4'chlorophenyl)isoxazole | 90 | $C_{15}H_{14}N_2O_4Cl_2$ | 50.4<br>50.5 | 3.9<br>4.0 | 7.8<br>8.0 |
| 64 | 3-[N(2'acetoxy dichloroethanoyl)N-ethylamino] 5-[(4'chlorophenyl)isoxazole | 102 | $C_{15}H_{13}N_2O_4Cl_3$ | 46.0<br>46.4 | 3.3<br>3.4 | 7.2<br>7.4 |
| 65 | 3-[N(hydroxyacetyl)N-ethylamino]5-(4-chlorophenyl isoxazole | 118 | $C_{13}H_{13}N_2O_3Cl$ | 55.6<br>55.8 | 4.6<br>4.6 | 10.0<br>10.0 |
| 66 | 3-[N-hydroxy(acetyl)N-ethylamino]4-chloro-5-(4"chlorophenyl)isoxazole | 104 | $C_{13}H_{12}N_2O_3Cl_2$ | 49.5<br>49.8 | 3.8<br>4.0 | 8.9<br>8.4 |
| 67 | 3-[methylamino)4-chloro-5-(3'4'dichlorophenyl) isoxazole | 143 | $C_{10}H_7N_2OCl_3$ | 43.2<br>43.9 | 2.5<br>2.7 | 10.1<br>10.3 |
| 68 | 3-[N(2'bromoethanoyl)N-methylamino]5-(3'4'di-chlorophenyl)isoxazole | 132 | $C_{12}H_9N_2O_2Cl_2Br$ | 39.6<br>40.1 | 2.5<br>2.5 | 7.7<br>7.7 |

TABLE I (continued)

| No | Compound | Melting Point °C | Analysis | C | H | N |
|---|---|---|---|---|---|---|
| 69 | 3-[N(2'acetoxythioethanoyl)N-ethylamino]-4-chloro-5(4'chlorophenyl)isoxazole | oil | $C_{15}H_{14}N_2O_3SCl_2$ | 48.3<br>48.3 | 3.8<br>3.8 | 7.5<br>7.7 |
| 70 | 3-[N-(2'ethylaminoethanoyl)N-ethylamino] 4-chloro-5-(4"chlorophenyl)isoxazole | 33 | $C_{15}H_{17}N_3O_2Cl_2$ | 52.6<br>53.8 | 5.0<br>5.1 | 12.3<br>12.3 |
| 71 | 3-[N-trichloroacetyl N-ethylamino] 4-bromo-5-(4'chlorophenyl)isoxazole | oil | $C_{13}H_9N_2O_2Cl_4Br$ | 34.9<br>35.3 | 2.0<br>2.1 | 6.3<br>6.2 |

TABLE II

| Exp. No. | Compound | Dose (ppm) | No. oöcysts in infection dose | survival[1] (A) | weight gain[2] (B) | cocci index (A + B) |
|---|---|---|---|---|---|---|
| 1 | No additive/ uninfected chickens | - | 0 | 100 % | 100 % | 200 |
| | Placebo | - | 50.000 | 100 | 66.1 | 166.1 |
| | 3 | 250 | 50.000 | 100 | 98.9 | 198.9 |
| | Nicarbazin | 125 | 50.000 | 100 | 105.9 | 205.9 |
| 2 | No additive/ uninfected chickens | - | 0 | 100 | 100 | 200 |
| | Placebo | - | 75.000 | 100 | 24 | 124 |
| | 3 | 250 | 75.000 | 100 | 66.4 | 166.4 |
| | 3 | 125 | 75.000 | 100 | 80 | 180 |
| | 6 | 250 | 75.000 | 100 | 71.8 | 171.8 |
| | Nicarbazin | 125 | 75.000 | 100 | 88.5 | 188.5 |
| 3 | No additive/ uninfected chickens | - | 0 | 100 % | 100 % | 200 |
| | Placebo | - | 100.000 | 100 | 44.2 | 144.2 |
| | 3 | 250 | 100.000 | 100 | 79.4 | 179.4 |
| | 3 | 125 | 100.000 | 100 | 60.8 | 160.8 |
| | 6 | 250 | 100.000 | 100 | 67.7 | 167.7 |
| | 6 | 125 | 100.000 | 100 | 51.1 | 151.1 |
| | Monensin | 125 | 100.000 | 100 | 70.8 | 170.8 |

TABLE II (Continued)

| Exp. No. | Compound | Dose (ppm) | No. oöcysts in infection dose | survival[1] (A) | weight gain[2] (B) | cocci index (A + B) |
|---|---|---|---|---|---|---|
| 4 | No additive/ uninfected chickens | - | 0 | 100 | 100 | 200 |
| | Placebo | - | 100.000 | 0 | 78 | 78 |
| | 3 | 250 | 100.000 | 100 | 94 | 194 |
| | 6 | 250 | 100.000 | 20 | 30 | 50 |
| | 14 | 250 | 100.000 | 100 | 167 | 267 |
| | Monensin | 125 | 100.000 | 0 | 106 | 106 |
| 5 | non-infected | - | - | | | 200 |
| | infected, untreated | - | 75,000 | | | 86.8 |
| | Monensin | 110 | 75,000 | | | 133.5 |
| | 3 | 125 | 75,000 | | | 155.0 |
| | 22 | 125 | 75,000 | | | 129.8 |
| | 31 | 125 | 75,000 | | | 166.2 |

Notes : 1 Deaths on day 5, 6 and 7 post infection.
2 Weight gain of uninfected control group is taken as 100 %

TABLE III

| Exp. No. | Compound | Dose (ppm) | No. oöcysts in infection dose | Eimeria Strain | survival[1] (A) | weight gain[2] (B) | cocci index (A + B) |
|---|---|---|---|---|---|---|---|
| 5 | No additive/ uninfected chickens | - | 0 | - | 100% | 100% | 200 |
| | placebo | - | 75.000 | E.tenella | 100 | 9.6 | 109.6 |
| | 22 | 150 | 75.000 | E.tenella | 100 | 86.8 | 186.8 |
| 6 | No additive/ uninfected chickens | - | 0 | - | 100% | 100% | 200 |
| | placebo | - | ? | E.necatrix | 100 | 86.2 | 186.2 |
| | 22 | 150 | ? | | 100 | 96.5 | 196.5 |

TABLE IV

| Compound No. | Invitro Evaluation against E. tenella | |
| --- | --- | --- |
| | Dose (ppm) | |
| | 100% Effective | >50% Effective |
| 36 | $10^{-3}$ | $10^{-6}$ |
| 41 | $10^{-2}$ | $10^{-3}$ |
| 39 | $10^{-3}$ | $10^{-5}$ |
| 40 | $10^{-2}$ | $10^{-5}$ |
| 42 | $10^{-3}$ | $10^{-3}$ |
| 46 | $10^{-2}$ | $10^{-5}$ |
| 42 | $10^{-2}$ | $10^{-4}$ |

K 1974 GBR

CLAIMS

1. Anticoccidial composition comprising an anticoccidially effective amount of a compound of the general formula I

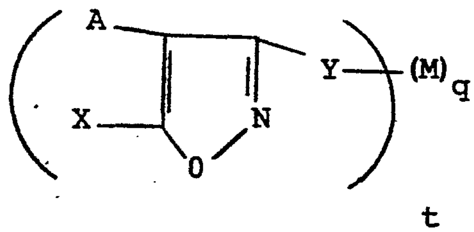

I

in which A represents hydrogen, Cl, Br, I, CN or $NO_2$, X represents a hydrogen atom or an optionally substituted alkyl, aralkyl, aryl, alkaryl, or a heterocyclic group and Y represents a nitrogen-containing heterocyclic group, or a group of general formula II

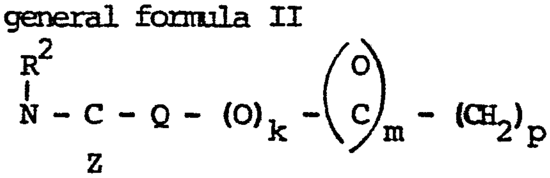

II

in which m is 1 or 0, Z is oxygen or sulphur, Q is hydrogen or 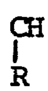

in which R is an alkyl group having 1-4 carbon atoms or a halogen atom, or Q is $(CH_2)_n$, n being 0-5 or Q is CHHal or $CH_2Hal$ wherein Hal is halogen, $R^2$ is hydrogen or an optionally substituted alkyl or cycloalkyl group, k is 0 or 1, p is 0-5, M is a heterocyclic group containing 1 or more nitrogen atoms or is, halogen, hydrogen, $NHR^1$, $SR^1$, $OR^1$, $NR^1$, $R^1$, N or $N^+$, $R^1$ being an optionally substituted

phenyl group, hydrogen or an alkyl group having 1-4 carbon atoms optionally substituted by -OH, -COOH or -COOalkyl, the alkyl group having 1-4 carbon atoms, q being 1 or 0, and t is 1-4 or when t=1 and q=0, Y represents a group of general formula III

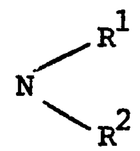

III

, in which $R^1$ and $R^2$, which can be the same or different, each represent a hydrogen atom or an optionally-substituted $C_2$-$C_6$ alkyl, cycloalkyl, alkenyl, aryl, alkaryl, aralkyl, acyl, carboxy, alkoxycarbonyl, alkylcarbonyl, dialkoxycarbonyl or sulphonyl group, or in which $R^1$ and $R^2$ together form a ring structure, which including the N atom is preferably a 5 or 6 membered ring structure or a salt thereof together with a veterinarily acceptable carrier.

2. Anticoccidial composition as claimed in claim 1 in which t=1, q=1, M is OH, and Y is a group of general formula II in which $R^2$ is an alkyl group having 1-4 carbon atoms, Z is oxygen and Q is $(CH_2)_n$ wherein n=0-3, k=0, m=1, and p=0.

3. Anticoccidial composition as claimed in any one of the claims 1-2 in which, when X represents an aralkyl, aryl, alkaryl or a substituted heterocyclic group, of which at least one of the substituents is an electron-withdrawing group.

4. Anticoccidial composition as claimed in any one of the claims 1-3 in which X represents a phenyl group substituted by at least chlorine or bromine.

5. Anticoccidial composition as claimed in any one of the claims 1-4 which comprises a compound of general formula I in which X is p-chlorophenyl, t=1, q=0 and Y is

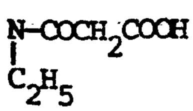

or

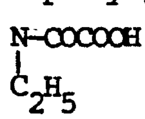

6. Poultry feed in which a compound of formula I or an anticoccidial composition as claimed in any one of the claims 1-5 has been incorporated.

7. Method for preparing a poultry feed as claimed in claim 6 which comprises mixing with or incorporating in, bird-edible material a compound of formula I or an anticoccidial composition as claimed in any one of the claims 1-5.

8. Method as claimed in claim 7 in which the anticoccidial composition is of the nature of a conventional kind of poultry premix.

9. Method for treating poultry infected by coccidia which comprises administering thereto, preferably in admixture with its feed a compound of formula I or an anticoccidial composition as claimed in any one of the claims 1-5.

10. Method for rearing birds for ultimate human consumption which comprises administering thereto a compound of formula I, or an anticoccidial composition as claimed in any one of the claims 1-5, or a poultry feed as claimed in claim 6.

11. Process for preparing compounds of formula I in which t=1, q=0 and X is a halo-substituted phenyl radical and Y is a mono- or dialkylaminoradical which comprises reacting a halo-substituted acetophenone with an alkyl isothiocyanate and methyliodide in the presence of sodiumhydride to form an intermediate of general formula IV

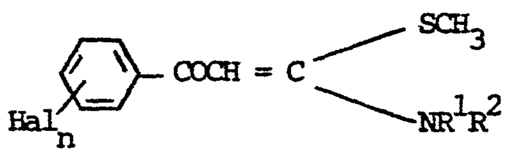

IV

in which Hal is chlorine or bromine, n=1-5, $R^1$ and $R^2$ having the same meaning as in the group of formula III, as defined in claim 2, which is thereafter converted to the isoxazole by reaction with hydroxylamine.

12. 5-(4'-chlorophenyl)-3-[ethylamino(N-carboxyethanoyl)]-isoxazole.

13. 5-(4'-chlorophenyl)-3-[ethylamino(N-carboxycarbonyl)]-isoxazole.
14. Novel compounds of formula I as defined in claim 1 other than those claimed in claims 12 and 13.
15. Anticoccidial composition as hereinbefore described with particular reference to the Examples.
16. Process for preparing compounds of formula I in which t=1, q=0 and X is a halo-substituted phenyl radical and Y is a mono- or dialkylaminoradical as hereinbefore described with particular reference to the Examples.